(19) Europäisches Patentamt
European Patent Office
Office européen des brevets

(11) **EP 4 424 666 A1**

(12) **EUROPEAN PATENT APPLICATION**

(43) Date of publication:
**04.09.2024 Bulletin 2024/36**

(21) Application number: **23159785.7**

(22) Date of filing: **02.03.2023**

(51) International Patent Classification (IPC):
***C07C 273/04*** (2006.01)  ***G01N 11/00*** (2006.01)

(52) Cooperative Patent Classification (CPC):
**C07C 273/04; G01N 9/002; G01N 9/36**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **CASALE SA**
**6900 Lugano (CH)**

(72) Inventors:
- **MARRONE, Leonardo**
  **21020 Mercallo (VA) (IT)**
- **BENEDETTI, Alberto**
  **22100 Como (IT)**
- **COLMEGNA, Giacomo**
  **6900 Massagno (CH)**

(74) Representative: **M. Zardi & Co S.A.**
**Via G. B. Pioda, 6**
**6900 Lugano (CH)**

(54) **METHOD FOR DETERMINING PARAMETERS OF A STREAM OF A UREA SYNTHESIS PROCESS**

(57) A method for determining at least one composition parameter of a urea-synthesis process stream comprising the step of measuring the density of said stream by means of a vibration density meter in contact with said stream and determining the ammonia/carbon dioxide molar ratio N/C of said stream on the basis of the density measured by said vibration density meter.

FIG. 1

**Description**

Field of application

The invention relates to measuring parameters of a urea-containing stream in a urea production process.

Prior art

Urea is produced industrially by reacting ammonia and carbon dioxide. An overview of the processes for the production of urea can be found in Ullmann's Encyclopedia of Industrial Chemistry, "Urea".

Urea is synthesized primarily in a urea synthesis reactor under high pressure and high temperature, typically at around 150 bar and 200 °C. In the reactor, ammonia and carbon dioxide form ammonium carbamate and dehydration of ammonium carbamate forms urea and water. The effluent of the reactor is an aqueous solution containing urea and unreacted ammonium carbamate; this solution is generally processed to recover the significant quantity of unconverted reagents contained therein. In a modern urea process, the reactor effluent is sent to a high-pressure stripper where gaseous ammonia and carbon dioxide are removed and sent to a high-pressure condenser. Stripping may be performed with the aid of gaseous ammonia or gaseous CO2 introduced in the stripper to act as a stripping medium.

The urea-containing solution from the stripper is sent to one or more recovery sections at a lower pressure, such as a low-pressure recovery section or a medium-pressure section followed by a low-pressure section. The recovery process generally includes thermal dissociation of ammonium carbamate still contained in the solution, separation of gaseous ammonia and CO2 and their condensation to form a recycle solution containing ammonium carbamate. Said recycle solution obtained in the recovery section is sent back to the synthesis loop, for example to the high-pressure condenser.

In a urea synthesis process, a number of items operating at high pressure, generally above 100 bar, can be identified. These items typically include the urea synthesis reactor, the high-pressure stripper, the high-pressure condenser and possibly a high-pressure scrubber. These items may be referred to as synthesis loop.

A proper control of the urea synthesis process requires to measure various parameters of the relevant process streams. A stream of particular interest is the aqueous urea solution effluent from the synthesis reactor. It is known that key parameters of a urea reactor are the N/C ratio and the H/C ratio, wherein N/C is the equivalent Ammonia / Carbon dioxide molar ratio and H/C is the equivalent Water / Carbon dioxide molar ratio.

The N/C can be calculated as follows:

$$\frac{N}{C} = \frac{2 mol_{Urea} + mol_{NH3}}{mol_{Urea} + mol_{CO2}}$$

The H/C can be calculated as follows:

$$\frac{H}{C} = \frac{mol_{H2O} - mol_{Urea}}{mol_{Urea} + mol_{CO2}}$$

It is broadly accepted that the liquid outlet of a reactor closely approaches the chemical equilibrium corresponding to a given outlet temperature and to a given inlet composition in terms of N/C and H/C. Several advantages are envisaged when the N/C ratio is kept within an optimal range, including a reduction in steam consumption, a reduction in the emission of unreacted ammonia and a maximization of urea production. An optimal N/C ratio may also contribute to the stable operation of the plant. There is therefore the interest to know the N/C and H/C of the reactor effluent in order to provide accurate control of the synthesis process. More generally, there is a need to measure directly or indirectly N/C and H/C at suitable locations of a urea plant, including typically the reactor outlet and possibly other locations. Unfortunately, this task is not easy particularly due to the highly corrosive properties and content of dissolved gasses of the urea-containing solution.

A first method of the prior art is off-line analysis of samples taken at selected locations of the plant. This method however introduces safety concerns in relation to manual extraction of samples and is intrinsically discontinuous, not allowing a real-time optimization of the plant.

Other methods determine the N/C indirectly on the basis of a known correlation between N/C and density. This is typically made with the use of Coriolis mass flow meters. Coriolis mass flow meters measure the mass flow rate and the density of the fluid. The N/C ratio is then estimated from a known relationship between the N/C and density, which may be determined experimentally or taken from the literature. Another method is described in JP-A-960341520.

The Coriolis mass flow meters still have some drawbacks. They require that a stream of the urea-containing medium is extracted for analysis and returned to the plant. To do so, dedicated expensive equipment such as a pump, an extraction line and a returning line are needed, particularly if the stream to be analyzed is extracted from a pressure vessel, such as the synthesis reactor.

A further drawback is that the urea stream may crystallize into the extraction line, especially during transitory events such as start-up and shutdown of the reactor. Therefore, a dedicated auxiliary system is required to perform a cleaning of the line in the event of crystallization. Further, the accuracy and especially reliability of this type of instrument in a urea environment is questionable.

Summary of the invention

The invention aims to overcome the above drawbacks of the prior art. In particular, the present invention aims to provide an effective method for estimating parameters of interest of urea synthesis process streams.

The above aim is reached with a method according to the claims. The invention is based on the use of a vibrating density meter to measure the density and possibly the viscosity of a target process stream of a urea synthesis process.

The density of the stream is a parameter of interest. The density may be of interest as such or as an indirect means to determine other parameters Particularly, in a urea-containing stream, the ratio N/C can be calculated as a function of the density with a good approximation. Measuring the viscosity in addition to the density gives further information about the stream and may be useful to determine the H/C ratio. Consequently, one or more composition parameters of great interest such as N/C and H/C can be computed with the invention.

A process stream of a urea synthesis process is understood as a stream containing at least ammonia, carbon dioxide and water. The process stream may contain urea, although certain process streams of interest may contain little or no urea. Process streams of noticeable interest include the reactor effluent, which is an aqueous solution of urea, and recycle carbamate stream. The process stream may be a liquid stream or a gaseous stream.

The process streams of interest are mixtures. In cases of practical interest, the target stream is at elevated temperature and pressure. Additionally, in cases of interest at least one of the components of the mixture may be above the critical temperature and/or above the critical pressure. The critical parameters are referred to the component in a pure state.

The invention is based on the surprising finding that a vibrating density meter can provide accurate and reliable density measurements also under the high temperature and high pressure found in the process streams a urea synthesis process, particularly in the urea synthesis section, and even if the target stream is a mixture wherein at least one component has a supercritical temperature and/or a supercritical pressure. The term of supercritical state is used to denote a urea process stream wherein at least one component is above at least one critical parameter.

Advantages of the invention include the following. A vibrating density meter can be installed directly on a pipe or on a vessel, such as the urea reactor. Accordingly, no sampling line or extraction line is required. Vibrating density meters can be manufactured from a variety of corrosion resistance alloys which make them particularly adapted to be used in corrosive urea environment. In addition, vibrating density meters can be manufactured weld-free to prevent the occurrence of localized corrosion.

Further advantages include that vibrating density meters do not pose safety concerns and gives a good accuracy, generally better than Coriolis flow meters.

Detailed description

The vibrating density meters are known in the art. A vibrating density meter includes a sensing element located in a chamber which can be filled with the tested fluid, so that the sensing element is immersed in the fluid. A vibration is imposed to the sensing element and, by detecting the mechanical response of the sensing element, the properties of the fluid such as density and viscosity can be determined. In a common embodiment, the sensing element is an elongated rod. If the fluid is in a gaseous state, the sensing element may have a feature to increase the contact surface with the fluid, such as an end flap for example.

Vibrating density meters are known for measuring the density of monophasic fluids such as liquids and gases. The previously known applications to liquids are restricted to liquids with low content of dissolved gas, such as crude oil or polymer compositions. The urea-containing streams of a urea synthesis process, particularly the synthesis reactor effluent, however, have a liquid phase with a significant amount of dissolved gas possibly in supercritical state. Prior to the present invention, it was generally believed that dissolved gas would alter the accuracy of a vibrating density meter due to formation of bubbles on the surface of the sensing element.

The invention is based on the very unexpected finding that a vibrating density meter gives accurate and repeatable measure on urea synthesis process streams and particularly on urea-containing liquid streams, such as the urea-containing aqueous solution as it is typically found at the outlet of a urea synthesis reactor. Without being bound by theory, it is believed that such unpredicted precision is due to the nearly critical or supercritical conditions of the urea-containing streams, wherein the physical properties of the liquid are similar to the properties of the gas. Therefore, the invention overcomes a strong and well rooted prejudice of the prior art.

A significant advantage of a vibrating density meter is that said meter can be installed directly to a urea synthesis reactor by means of a flange. Accordingly, no sampling line or extraction line is required to perform the measurements.

It is broadly accepted that physical properties such as density and viscosity are dependent on temperature, pressure, and composition. The impact of pressure can be usually neglected for liquids whereas it may not be negligible for gases. At a given temperature, it was experimentally found that the density of a urea-containing liquid stream subject to chemical equilibrium is mainly dependent on the N/C although a minor dependence on the H/C was also observed.

The main univocal correlation between the density of the urea-containing liquid stream subject to chemical equilibrium and the N/C ratio of said stream can be determined experimentally or from the literature data. Then the N/C can be estimated on the basis of the measured density and of said correlation.

In an interesting embodiment, the method further includes to measure the viscosity of the urea synthesis process stream by means of said vibration-type density meter. The density and the viscosity measured together with the temperature and possibly the pressure of the urea synthesis process stream can then be used to determine an additional composition parameter of the stream, preferably the H/C ratio. Measuring the pressure is appropriate if the process stream is gaseous. For a liquid stream, the knowledge of the temperature is generally sufficient.

Particularly preferably the target stream is a urea-containing liquid stream and more preferably a urea-containing liquid reaction effluent, namely the effluent of a urea synthesis environment, such as a urea reactor. Preferably the temperature of said urea-containing reaction effluent is measured at the outlet of the synthesis reactor. The temperature of said urea-containing reaction effluent is preferably comprised between 170 to 230 °C, more preferably 180 to 210 °C, particularly preferably 180 to 190 °C.

In an embodiment of practical interest, said target urea-containing liquid stream is in a nearly critical or supercritical state. In a preferred embodiment at least one of the temperature and pressure is supercritical (namely above the critical value) or both temperature and pressure are supercritical.

In addition to the above-mentioned N/C and H/C, a further parameter of interest is the ratio U/C of moles of urea over moles of carbon which is determined as follows:

$$\frac{U}{C} = \frac{mol_{urea}}{mol_{Urea} + mol_{CO2}}$$

In an embodiment, the invention provides that N/C, H/C and U/C are calculated on the basis of the temperature, density and viscosity of a reaction effluent, as well as constant of equilibrium in the reactor $K_{eq}$. The knowledge of the temperature, density and viscosity, as well as knowledge of $K_{eq}$, allows to compute N/C, H/C and U/C on the basis of the following system of equations:

$$\rho = f_1\left(T, \frac{N}{C}, \frac{H}{C}\right) \qquad (1)$$

$$\mu = f_2\left(T, \frac{N}{C}, \frac{H}{C}\right) \qquad (2)$$

$$Keq = f_3\left(\frac{N}{C}, \frac{H}{C}, \frac{U}{C}, T\right) \qquad (3)$$

Wherein $\rho$ (rho) denotes the density, $\mu$ (mu) denotes the viscosity, T is the temperature, $K_{eq}$ is the constant of equilibrium of the urea conversion reaction. Preferably the equilibrium constant is determined from literature data. The functions $f_1$, $f_2$ and $f_3$ can be obtained from literature data or from fitting of experimental data thus they are known parameters. Knowing the values of T, $\rho$, $\mu$ and equilibrium constant, the above equations (1), (2), (3) form a system of three equations with three unknowns (N/C, H/C, U/C) which can be solved.

Therefore, an embodiment of the invention may include:

determining the first function $f_1$ which represents the density of the urea containing stream as a function of: the temperature of the urea containing stream, the N/C ratio and the H/C ratio;

determining the second function $f_2$ which represents the viscosity of the urea containing medium as a function of temperature, N/C, and H/C;

determining the third function fa which represents the equilibrium constant of the urea conversion reaction, as a function of N/C, H/C, U/C and of the temperature measured;

solving the above system of equations (1), (2) and (3) to find the values of N/C, H/C, U/C. The system may be solved numerically according to known techniques.

In a particularly interesting application, the target stream is a urea-containing reaction effluent; the method includes determining the density and the viscosity of the target stream using the vibration density meter; measuring the temperature of the target stream at the outlet of a urea synthesis reactor; from the knowledge of the equilibrium constant of the urea synthesis reaction computing the values of N/C, H/C, U/C from the system of equations as above explained.

This method is further elucidated in the example reported below. An example of the functions $f_1$, $f_2$ and $f_3$ is also given.

The method of the invention may be implemented in a suitable control system. An aspect of the invention is a control system for an equipment of a urea synthesis plant, particularly for a urea synthesis reactor, wherein the control system is configured to implement a method according to any of the embodiments described above. The control system may be configured to monitor an input stream or output stream of a specific equipment, a preferred application being the effluent of a urea synthesis reactor. A preferred application of the invention is a control system of a urea synthesis reactor.

The method of the invention can be performed continuously on a running urea synthesis process, so that the target stream is monitored in real time. For example, one or more of the above-mentioned parameters such as N/C, H/C, U/C of the target stream can be determined in real-time.

A preferred vibration density meter is a rod-type meter wherein the sensing element has the shape of an elongate rod. A rod-type meter may include a single rod or a plurality of rods. The rod can be either cylindrical or with additional wings / flaps or other features to increase the wet surface depending on the application.

The sensing element is manufactured preferably with a corrosion-resistant material. Preferably said corrosion resistant material is pure titanium or a titanium alloy. Alternatively, the sensing element can be manufactured in a high-nickel austenitic stainless steel or a duplex stainless steel. Preferred materials include steel 25-22-2, Superduplex UNS 32906, 316L Urea-grade or Safurex®.

A very interesting application of the invention concerns the effluent of a urea synthesis reactor. The reactor effluent is essentially a mixture of water, urea, ammonium carbamate, other salts such as ammonium carbonate and ammonium bicarbonate, free ammonia and CO2 dissolved in the liquid. In certain embodiments the composition of the reactor effluent is modeled in terms of equivalent moles of CO2, NH3 and H2O. Said equivalent moles account for the content of Carbon, Nitrogen and Hydrogen in the stream. The equivalent moles of CO2 are moles of CO2 which contains the same atoms of Carbon as the reactor effluent and similarly the equivalent moles of NH3 and H2O correspond to the content in Nitrogen and Hydrogen. By referring to this model, the composition of the stream can be easily represented in terms of N/C, H/C, U/C. this provides a simplified but effective approach for controlling the process, for example for controlling a urea reactor.

Example

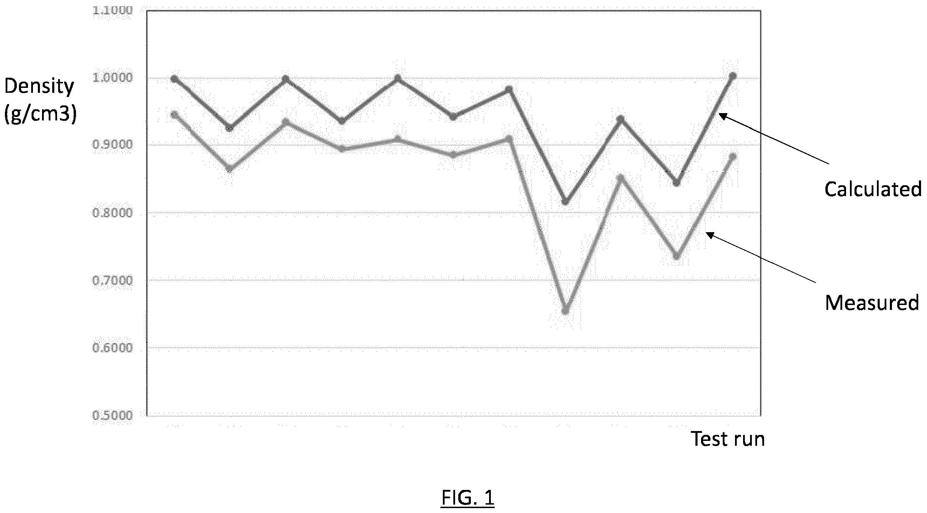

Fig. 1 is a plot showing experimental density measured in accordance with the invention Vs. density data calculated using a dependable model implemented with the process simulation software UniSim®.

Fig. 1 reports data obtained with more than 30 runs performed on a test facility in an autoclave under different operating conditions, with N/C ranging from around 2.9 to around 6.2 and temperature from 185 °C to 195 °C.

Fig. 1 compares density measured in accordance with the invention with valued predicted with the simulation software, showing that experimental measures were consistent with the model. Further it was observed that the density signal was very stable over time and excellent reproducibility was found. No reliability issues were detected.

The plot of Fig. 1 demonstrates that a vibration density meter provides a reliable measure of the density of a urea-containing stream under urea-forming conditions of temperature and pressure. From the density, the N/C can be determined.

Within the range of interest, the N/C can be assumed to have a linear relationship with the density. Therefore N/C can be calculated as:

$$N/C = a\, \rho^{\,b}$$

wherein $\rho$ is the density (kg/cm$^3$), **a** and **b** are coefficient determined experimentally. For example, for a urea-containing solution at equilibrium at 185 °C and N/C between around 3.0 and 4.5 the applicant has found experimental data which are well fitted by coefficients **a** = 1.6519 and **b** = -0.517. Other correlations may be found in the literature.

The following is an example of how to determine the parameters N/C, H/C e U/C in a urea reactor effluent. The method is based on a density-viscosity-equilibrium model making use of a density equation, viscosity equation and equilibrium equation. Input data are density and viscosity measured by a vibration density meter according to the invention and temperature measured by a suitable temperature probe. On the basis of the three equations of the model, the parameters N/C, H/C and U/C can be calculated.

Density equation:

$$\rho(N/C, H/C, T) =$$
$$= \begin{cases} \rho_{185} = a_1 + a_2\, N/C + a_3\, H/C + a_4\, N/C \cdot H/C & T = 185°C \\ \dfrac{\rho_{195} - \rho_{185}}{195 - 185}(T - 185) + \rho_{185} & 185 < T < 195°C \\ \rho_{195} = b_1 + b_2\, N/C + b_3\, H/C + b_4\, N/C \cdot H/C & T = 195°C \end{cases}$$

wherein:

$$(a_1;\ a_2;\ a_3;\ a_4) = (1.772;\ -2.6323\text{e-}01;\ -7.4272\text{e-}01;\ 2.2374\text{e-}01)$$

$$(b_1;\ b_2;\ b_3;\ b_4) = (1.1836;\ -7.0823\text{e-}02;\ -2.4142\text{e-}01;\ 4.6252\text{e-}02)$$

The above density equation and coefficients fit a temperature range of 185 to 195 °C; N/C in the range 3 to 3.5 and H/C in the range 0.5 to 1.0. The coefficients are given in scientific notation wherein the digits after the symbol "e" denotes powers of ten.

Viscosity equation:

$$\mu\left(\frac{N}{C}, \frac{H}{C}, T\right) =$$
$$= \begin{cases} \mu_{185} = c_1 + c_2\, N/C + c_3\, H/C + c_4\, N/C \cdot H/C & T = 185°C \\ \dfrac{\mu_{195} - \mu_{185}}{195 - 185}(T - 185) + \mu_{185} & 185 < T < 195°C \\ \mu_{195} = d_1 + d_2\, N/C + d_3\, H/C + d_4\, N/C \cdot H/C & T = 195°C \end{cases}$$

wherein:

$$(c_1;\ c_2;\ c_3;\ c_4) = (5.2958\text{e-}01;\ 4.8882\text{e-}02;\ 4.4359;\ -9.3751\text{e-}01)$$

$$(d_1;\ d_2;\ d_3;\ d_4) = (1.1836;\ -7.0823\text{e-}02;\ -2.4142\text{e-}01;\ 4.6252\text{e-}02)$$

The viscosity equation and coefficients fit a temperature range of 185 to 195 °C; N/C in the range 3 to 3.5 and H/C in the range 0.5 to 1.0. The coefficients are given in scientific notation as in the previous case.

Equilibrium:

The equilibrium constant is intended in relation to the reaction of synthesis of urea:

$$2NH_3 + CO_2 = Urea + H_2O$$

and has the form:

$$K_{eq} = \frac{x_U \, x_{H2O}}{x_{CO2} \, x_{NH3}^2} = \frac{U/C \, (N/C + U/C)(1 + N/C + H/C - U/C)}{\left(1 - \frac{U}{C}\right)\left(\frac{N}{C} - 2\frac{U}{C}\right)^2} =$$

$$= h(N/C, H/C, U/C, T)$$

The function $h(N/C, H/C, U/C, T)$ can be determined by fitting experimental data.

Reference can also be made to the following paper about the equilibrium in a urea reactor: Inoue, Kanai, Otsuka, "Equilibrium of Urea Synthesis", Bulletin of the Chemical Society of Japan, vol. 45, 1339-1345.

## Claims

1. Method for monitoring a urea synthesis process stream, in a urea synthesis process, comprising the step of measuring the density of said urea process synthesis stream by means of a vibration density meter in contact with said stream.

2. Method according to claim 1 including the step of determining at least one composition parameter of said urea synthesis process stream on the basis of the density measured by the vibration density meter.

3. Method according to claim 2 comprising:

   a) measuring the density of said urea process synthesis stream by means of said vibration density meter;
   b) determining the N/C ratio of said stream on the basis of the density measured at step a), wherein N/C is the ratio between the equivalent ammonia and carbon dioxide contained in the stream according to the formula:

$$\frac{N}{C} = \frac{2mol_{Urea} + mol_{NH3}}{mol_{Urea} + mol_{CO2}}$$

4. Method according to any of the previous claims wherein the urea synthesis process stream is a liquid stream, preferably a urea-containing liquid stream and more preferably an aqueous solution of urea effluent from a urea synthesis environment.

5. Method according to any of the previous claims including:

   providing a correlation between the density of the urea synthesis process stream and the N/C of said stream;
   determining the N/C of said stream on the basis of the measured density and of said correlation.

6. Method according to any of the previous claims further including the step of measuring the viscosity of the stream by means of said vibration-type density meter;
   the method further includes determining the H/C ratio of the stream wherein H/C is defined according to the following formula:

$$\frac{H}{C} = \frac{mol_{H2O} - mol_{Urea}}{mol_{Urea} + mol_{CO2}}$$

7. Method according to any of the previous claims wherein the urea synthesis process stream is a urea-containing stream effluent from a synthesis environment where reagents and product of the urea synthesis reaction are at chemical equilibrium, the method including:

determining a first function which represents the density of the stream as a function of temperature, N/C and H/C;
determining a second function which represents the viscosity as a function of temperature, N/C, H/C and U/C, wherein U/C is a ratio of moles of urea over moles of carbon;
determining a third function which represents the equilibrium constant of the urea conversion reaction in the urea-containing stream, as a function of N/C, H/C, U/C and temperature;
wherein the density and the viscosity are determined by said vibration density meter; the temperature is measured and the equilibrium constant is calculated;
computing the values of N/C, H/C, U/C from said first, second and third function using the density, viscosity, temperature and equilibrium as input data.

8. Method according to any of the previous claims, wherein the method is performed continuously on a running urea synthesis process, so that said urea synthesis process stream is monitored in real time.

9. Method according to any of the previous claims, including the step of representing the composition of said urea synthesis process stream in terms of equivalent moles of $CO_2$, equivalent moles of $NH_3$ and equivalent moles of water.

10. Method according to any of the previous claims wherein said vibration density meter is a rod-type meter wherein the meter includes at least one sensing element in the form of an elongated rod.

11. Method according to any of the previous claims wherein said urea synthesis process stream is a mixture wherein at least one component of the mixture has a temperature and/or pressure above critical value.

12. A control system for an equipment of a urea synthesis plant, particularly for a urea synthesis reactor, wherein the control system is configured to implement a method according to any of the previous claims.

FIG. 1

## EUROPEAN SEARCH REPORT

Application Number

EP 23 15 9785

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 728 783 A1 (TOYO ENGINEERING CORP [JP]) 6 December 2006 (2006-12-06) * claim 1 * * paragraphs [0002], [0007], [0039] * | 1-12 | INV. C07C273/04 G01N11/00 |
| X | WO 2021/261999 A1 (STAMICARBON [NL]) 30 December 2021 (2021-12-30) * claim 1 * * page 18, line 32 – page 19, line 27 * * page 19, line 17 * * page 24, line 11 – line 18 * | 1-12 | |
| X | EP 0 114 442 A1 (UNIE VAN KUNSTMESTFAB BV [NL]) 1 August 1984 (1984-08-01) * claims 1-8 * * page 2, lines 11-16 * | 1-12 | |
| X,D | JP H10 182586 A (TOYO ENGINEERING CORP) 7 July 1998 (1998-07-07) * paragraph [0008]; claims 1-5 * | 1-12 | |

TECHNICAL FIELDS SEARCHED (IPC)

C07C
G01N

The present search report has been drawn up for all claims

| Place of search | Date of completion of the search | Examiner |
|---|---|---|
| The Hague | 18 August 2023 | Seitner, Irmgard |

EPO FORM 1503 03.82 (P04C01)

## ANNEX TO THE EUROPEAN SEARCH REPORT
## ON EUROPEAN PATENT APPLICATION NO.

EP 23 15 9785

18-08-2023

| Patent document cited in search report | | | Publication date | Patent family member(s) | | | Publication date |
|---|---|---|---|---|---|---|---|
| EP 1728783 | A1 | | 06-12-2006 | EP | 1728783 | A1 | 06-12-2006 |
| | | | | JP | 4928740 | B2 | 09-05-2012 |
| | | | | JP | 2006335653 | A | 14-12-2006 |
| | | | | US | 2006270872 | A1 | 30-11-2006 |
| WO 2021261999 | A1 | | 30-12-2021 | AU | 2021296691 | A1 | 19-01-2023 |
| | | | | CA | 3186903 | A1 | 30-12-2021 |
| | | | | CN | 115916745 | A | 04-04-2023 |
| | | | | EP | 4168389 | A1 | 26-04-2023 |
| | | | | JP | 2023526689 | A | 22-06-2023 |
| | | | | US | 2022274916 | A1 | 01-09-2022 |
| | | | | WO | 2021261999 | A1 | 30-12-2021 |
| EP 0114442 | A1 | | 01-08-1984 | AT | E30192 | T1 | 15-10-1987 |
| | | | | CA | 1211297 | A | 16-09-1986 |
| | | | | EP | 0114442 | A1 | 01-08-1984 |
| | | | | ES | 8501882 | A1 | 01-12-1984 |
| | | | | IN | 161337 | B | 14-11-1987 |
| | | | | JP | S59133451 | A | 31-07-1984 |
| | | | | NL | 8204979 | A | 16-07-1984 |
| | | | | US | 4572830 | A | 25-02-1986 |
| | | | | YU | 249483 | A | 30-04-1986 |
| JP H10182586 | A | | 07-07-1998 | JP | 3270700 | B2 | 02-04-2002 |
| | | | | JP | H10182586 | A | 07-07-1998 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 960341520 A

**Non-patent literature cited in the description**

- Urea. Ullmann's Encyclopedia of Industrial Chemistry

- **INOUE ; KANAI ; OTSUKA.** Equilibrium of Urea Synthesis. *Bulletin of the Chemical Society of Japan,* vol. 45, 1339-1345